# EUROPEAN PATENT APPLICATION

(11) **EP 4 799 566 A1**
(43) Date of publication of application: **02.09.2026**
(21) Application number: 26155709.4
(22) Date of filing: 02.02.2026
(51) Int. Cl.: A61B 5/145, A61B 5/00

(54) **ELECTRONIC DEVICE AND BLOOD GLUCOSE DATA MANAGEMENT METHOD THEREOF**

(30) Priority: 27.02.2025 KR 20250026121
(71) Applicant: I-SENS, INC., Seoul 06646 (KR)
(72) Inventor: MOK, Jin Won, 06646 Seoul (KR); SEO, Jung Hee, 06646 Seoul (KR); KIM, Hae Na, 06646 Seoul (KR)
(74) Representative: BCKIP Part mbB

(57) **Abstract**

Provided is a blood glucose data management method including identifying first blood glucose data based on a blood glucose sensor, identifying a verification dataset corresponding to the first blood glucose data, estimating whether at least a portion of the blood glucose sensor is detached from a user's body based on a comparison between a pattern corresponding to the verification dataset and a reference pattern, and suspending output of the first blood glucose data if detachment is estimated.

## Description

### CROSS-REFERENCE TO RELATED APPLICATION(S)

This application claims the benefit of Korean Patent Application No. 10-2025-0026121, filed on February 27, 2025, in the Korean Intellectual Property Office.

### FIELD OF THE INVENTION

Example embodiments relate to an electronic device and a blood glucose data management method thereof.

### BACKGROUND

The continuous glucose monitoring system (CGMS) is a device that monitors blood glucose changes in diabetic patients in real time. It is widely used recently due to its convenience, allowing patients to measure glucose concentration in the interstitial fluid beneath the skin via a percutaneously inserted sensor without the need for blood sampling.

While CGMS offers many advantages, one drawback is that if the glucose sensor's attachment becomes unstable or the sensor detaches from the patient's skin, it can potentially produce distorted or unreliable blood glucose readings. Such risks leading to incorrect treatment decisions and could compromise patient safety.

Therefore, reliably detecting sensor detachment is critically important. However, sensor detachment often occurs without clear physical symptoms, making it difficult to detect through simple adhesion checks alone.

Furthermore, due to the nature of CGMS, which continuously measures blood glucose, the ability to statistically manage blood glucose readings is fundamentally provided and recognized as an important feature for users. From this perspective, the question of how to statistically process and manage data obtained from a detached sensor is an important consideration for improving the user experience. Nevertheless, discussion on this topic has not yet been sufficiently developed.

### SUMMARY OF THE INVENTION

An aspect provides an electronic device and a blood glucose data management method thereof, and more particularly, a method for detecting detachment of a blood glucose sensor based on a blood glucose pattern and managing blood glucose data obtained from the detached sensor.

The technical aspects of the present disclosure are not limited to those mentioned above, and other technical aspects can be inferred from the following example embodiments.

According to an aspect, there is provided a blood glucose data management method of an electronic device including identifying first blood glucose data based on a blood glucose sensor, identifying a verification dataset corresponding to the first blood glucose data, estimating whether at least a portion of the blood glucose sensor is detached from a user's body based on a comparison between a pattern corresponding to the verification dataset and a reference pattern, and suspending output of the first blood glucose data for the user if detachment is estimated.

In an example embodiment of the present disclosure, the blood glucose data management method may further include checking blood glucose data periodically based on the blood glucose sensor percutaneously inserted into the user's body before the identifying of the first blood glucose data based on the blood glucose sensor. The first blood glucose data may be measured by the blood glucose sensor during the next measurement cycle of the blood glucose data that is periodically checked.

Furthermore, in an example embodiment of the present disclosure, the identifying of the verification dataset corresponding to the first blood glucose data may include identifying at least one preceding blood glucose data whose blood glucose measurement sequence is consecutive to the first blood glucose data, and identifying the verification dataset including the first blood glucose data and the at least one preceding blood glucose data in a time-series alignment.

Furthermore, in an example embodiment of the present disclosure, the estimating of whether at least a portion of the blood glucose sensor is detached may include identifying a plurality of time-series aligned blood glucose data included in the verification dataset, determining whether a pattern of the plurality of blood glucose data corresponds to at least one of a first reference pattern and a second reference pattern included in the reference pattern, and estimating detachment of at least a portion of the blood glucose sensor if the pattern of the plurality of blood glucose data corresponds to at least one of the first and second reference patterns.

Furthermore, in an example embodiment of the present disclosure, the first reference pattern may include a pattern in which, in two blood glucose data points with consecutive blood glucose measurement orders among the plurality of blood glucose data, the subsequent blood glucose data decreases by more than a first threshold compared to the preceding blood glucose data.

Furthermore, in an example embodiment of the present disclosure, the first reference pattern may further include a pattern in which the slope between two blood glucose data points whose blood glucose measurement orders are later than that of the preceding blood glucose data among the plurality of blood glucose data is less than 0.

Furthermore, in an example embodiment of the present disclosure, the second reference pattern may include a pattern in which at least one blood glucose data among the plurality of blood glucose data is equal to or less than a second threshold.

Furthermore, in an example embodiment of the present disclosure, the estimating of whether at least a portion of the blood glucose sensor is detached may include adjusting at least one threshold related to the reference pattern based on the user's medical history information.

Furthermore, in an example embodiment of the present disclosure, the adjusting of at least one threshold related to the reference pattern may include identifying the medical history information indicating that the user has type 1 diabetes, and adjusting the first threshold of the first reference pattern related to the amount of decrease in the subsequent blood glucose data relative to the preceding blood glucose data among two consecutive blood glucose measurement sequences so that the first threshold increases.

Furthermore, in an example embodiment of the present disclosure, the adjusting of at least one threshold related to the reference pattern may include identifying the medical history information indicating that the user has a history of hypoglycemic shock, and adjusting the second threshold of the second reference pattern related to the size of at least one blood glucose data among the plurality of blood glucose data included in the verification dataset so that the second threshold is reduced.

Furthermore, in an example embodiment of the present disclosure, the blood glucose data management method may further include identifying second blood glucose data that has increased compared to the first blood glucose data based on the blood glucose sensor, releasing the suspension in outputting the first blood glucose data, and outputting the first blood glucose data to the user normally.

Furthermore, in an example embodiment of the present disclosure, the blood glucose data management method may further include, before the identifying of the second blood glucose data that has increased compared to the first blood glucose data based on the blood glucose sensor and after the releasing of the suspension in outputting the first blood glucose data, checking a flag related to compression noise set for the first blood glucose data, controlling the blood glucose sensor so that the second blood glucose data is measured earlier than the default cycle. The outputting of the first blood glucose data to the user may include outputting a notification related to compression noise to the user.

Furthermore, in an example embodiment of the present disclosure, the blood glucose data management method may further include determining whether the number of consecutive blood glucose data whose output has been suspended corresponds to a first reference number, and disconnecting the connection to the blood glucose sensor if the number of consecutive blood glucose data whose output has been suspended corresponds to the first reference number.

Furthermore, in an example embodiment of the present disclosure, the blood glucose data management method may further include identifying a new blood glucose sensor that replaces the blood glucose sensor when the connection to the blood glucose sensor is disconnected, identifying blood glucose data based on the new blood glucose sensor, identifying estimated blood glucose data for at least a portion of the consecutive blood glucose data whose output has been suspended by performing interpolation or extrapolation using the identified blood glucose data based on the new blood glucose sensor, and outputting the estimated blood glucose data to the user.

Furthermore, in an example embodiment of the present disclosure, the blood glucose data management method may further include outputting a notification regarding sensor detachment to the user.

Furthermore, in an example embodiment of the present disclosure, the outputting of the notification regarding sensor detachment to the user may include identifying a terminal of the user's caregiver, and outputting the notification related to sensor detachment to the terminal of the user's caregiver.

Furthermore, in an example embodiment of the present disclosure, the notification regarding sensor detachment may be different from at least a portion of a measurement notification, a hypoglycemia notification, and a hyperglycemia notification of the blood glucose sensor in terms of at least a portion of the sound volume, vibration intensity, and notification type.

Furthermore, in an example embodiment of the present disclosure, the blood glucose data management method may further include identifying a request for output of a blood glucose graph by the user, deactivating a portion of the blood glucose graph corresponding to blood glucose data whose output is suspended, including the first blood glucose data, and outputting the blood glucose graph to the user, with the portion corresponding to the blood glucose data whose output is suspended being deactivated. The deactivation may include at least a portion of blurring of points on the blood glucose graph, non-responsiveness to clicks, and displaying indication of output suspension.

According to another aspect, there is provided an electronic device including a processor and a memory storing one or more instructions. The processor is, by performing the one or more instructions, configured to identify first blood glucose data based on a blood glucose sensor, identify a verification dataset corresponding to the first blood glucose data, estimating whether at least a portion of the blood glucose sensor is detached from a user's body based on a comparison between a pattern corresponding to the verification dataset and a reference pattern, and suspending output of the first blood glucose data for the user if detachment is estimated.

According to yet another aspect, there is provided a non-transitory computer-readable recording medium having a program for executing the above-described blood glucose data management method on a computer recorded thereon.

Specific details of other example embodiments are included in the detailed description and drawings.

Additional aspects of example embodiments will be set forth in part in the description which follows and, in part, will be apparent from the description, or may be learned by practice of the disclosure.

### BRIEF DESCRIPTION OF THE DRAWINGS

These and/or other aspects, features, and advantages of the disclosure will become apparent and more readily appreciated from the following description of example embodiments, taken in conjunction with the accompanying drawings of which:
FIG. 1 is a diagram illustrating the interconnection between an electronic device managing blood glucose data, a blood glucose sensor, a server, and a caregiver terminal according to an example embodiment;
FIG. 2 is a flowchart illustrating a blood glucose data management method according to an example embodiment;
FIG. 3A is a diagram showing an example where the pattern of a verification dataset corresponds to the first reference pattern;
FIG. 3B is a diagram showing another example where the pattern of a verification dataset corresponds to the first reference pattern;
FIG. 3C is a diagram showing an example where the pattern of a verification dataset does not correspond to the first reference pattern;
FIG. 4A is a diagram showing an example where a notification related to sensor detachment is displayed via a notification window according to an example embodiment;
FIG. 4B is a diagram showing an example where a notification related to sensor detachment is displayed via a banner according to an example embodiment;
FIG. 5A is a diagram showing another example where a notification related to sensor detachment is displayed via a notification window according to an example embodiment;
FIG. 5B is a diagram showing another example where a notification related to sensor detachment is displayed via a banner according to an example embodiment;
FIG. 6A is an example diagram of a blood glucose graph where blood glucose data whose output has been suspended is deactivated according to an example embodiment;
FIG. 6B is an example diagram showing a user interface (UI) when the blood glucose data whose output has been suspended and thus deactivated is clicked according to an example embodiment; and
FIG. 7 is a block diagram of an electronic device according to an example embodiment.

### DETAILED DESCRIPTION OF EXEMPLARY EMBODIMENTS

The terms used in example embodiments have been selected as general terms that are currently widely used as possible while taking functions in the present disclosure into consideration, but these may vary according to the intention of those skilled in the art, a precedent, the emergence of new technologies, and the like. In addition, in certain cases, there are terms arbitrarily selected by the applicant, and in this case, the meaning will be described in detail in the corresponding description. Therefore, the terms used in the present disclosure should be defined based on the meaning of the term and the whole contents of the present disclosure, not just the name of the term.

Throughout the specification, when it is stated that a part "comprises" or "includes" a certain component, it means that other components may further be included, and it does not preclude other components, unless otherwise stated.

The expression "at least one of A, B, and C" may indicate the following meaning including: A alone; B alone; C alone; both A and B together; both A and C together; both B and C together; or all three of A, B, and C together.

The "terminal" mentioned herein may be implemented as a computer or a portable terminal that may access a server or other terminal through a network. Here, the computer includes, for example, a notebook, a desktop, a laptop, and the like, equipped with a web browser, and the portable terminal is, for example, a wireless communication device that guarantees portability and mobility, which may include all kinds of handheld-based wireless communication device including communication-based terminals such as IMT (International Mobile Telecommunication), CDMA (Code Division Multiple Access), W-CDMA (W-Code Division Multiple Access), LTE (Long Term Evolution), smartphones, tablet PCs, and the like.

In the following, with reference to the accompanying drawings, example embodiments of the present disclosure will be described in detail so that those of skilled in the art to which the present disclosure pertains may easily implement them. However, the present disclosure may be implemented in various different forms and is not limited to the example embodiments described herein.

Hereinafter, example embodiments of the present disclosure will be described in detail with reference to the drawings.

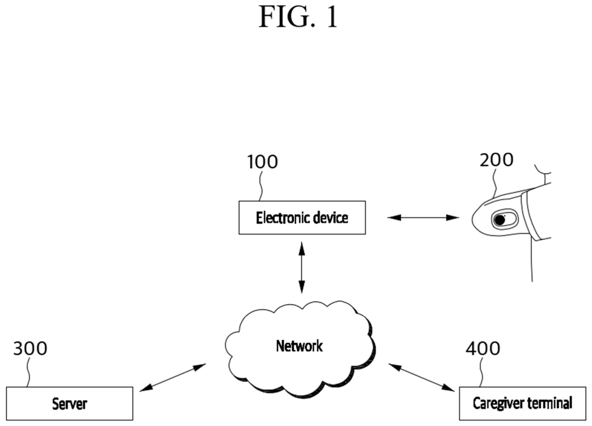

FIG. 1 is a diagram illustrating the interconnection between an electronic device managing blood glucose data, a blood glucose sensor, a server, and a caregiver terminal according to an example embodiment.

Referring to FIG. 1, an electronic device 100 may operate in conjunction with a blood glucose sensor 200, a server 300, and a caregiver terminal 400. Meanwhile, FIG. 1 shows only the components related to this example embodiment. Therefore, those skilled in the art related to this example embodiment will understand that other general-purpose components may be included in addition to the components shown in FIG. 1.

The electronic device 100 is a device that configures and provides various pieces of information. The electronic device 100 may provide the configured information as a web page or application screen, or provide it in a form that may be displayed as a web page or application screen on a receiving terminal. According to an example embodiment, the electronic device 100 may correspond to a terminal carried by a user, such as a smartphone or tablet, but is not limited thereto. The electronic device 100 may be connected to the blood glucose sensor 200 via a wired or wireless communication method. It may also be connected to the server 300 and the caregiver terminal 400 via a network.

The blood glucose sensor 200 may be percutaneously inserted into the user's body to measure glucose concentration in the user's interstitial fluid. For example, the blood glucose sensor 200 may include a microelectrode and an enzyme layer that are percutaneously inserted into the user's body, which may indirectly measure the glucose concentration in the interstitial fluid by detecting the flow of electrons resulting from the chemical reaction of glucose oxidase occurring in the enzyme layer, that is, the current value, as biological data via the microelectrode. However, the blood glucose sensor is not limited to operating on this principle. Any sensor method capable of measuring glucose concentration in the interstitial fluid may be applied to the blood glucose sensor 200 of this disclosure. When the blood glucose sensor 200 operates as described above, the blood glucose sensor 200 may be connected to the electronic device 100 via a wired or wireless communication method as previously mentioned and periodically transmit the measured biological data to the electronic device 100. According to an example embodiment, the electronic device 100 may compute blood glucose data by processing such biological data. For example, the electronic device 100 may perform the computation based on a mapping relationship between the biological data and the blood glucose data, but is not limited thereto. In the following description, detachment of at least a portion of the blood glucose sensor 200 may mean that all or part of the portion that must be percutaneously inserted into the user's body has detached and is exposed to air.

According to an example embodiment, the blood glucose sensor 200 bundles multiple biometric data measurements taken at short intervals, i.e., multiple current values, and transmits them to the electronic device 100 as a value representing a larger interval encompassing the short intervals. The electronic device 100 may then determine blood glucose data for the larger interval based on the transmitted value. For example, the blood glucose sensor 200 may measure biological data every 10 seconds and transmit the biological data measured every 10 seconds to the electronic device 100 grouped in 5-minute intervals. The electronic device 100 may calculate blood glucose data for the corresponding 5-minute cycle based on the average value of the biometric data measured every 10 seconds or various other representative values determined by other methods. Of course, the method of measuring blood glucose data is not limited to the above description. For example, blood glucose data could be measured by first measuring a current value, then computing the blood glucose data within the blood glucose sensor 200 itself, and subsequently transmitting it to the electronic device 100. Various methods by which the electronic device 100 measures blood glucose data based on the blood glucose sensor 200, other than the methods described above, may be encompassed by the present disclosure.

The server 300 is connected to the electronic device 100 via a network, enabling it to perform operations such as long-term backup of the user's blood glucose data collected by the electronic device 100 and providing the electronic device 100 with backed-up historical blood glucose data.

The caregiver terminal 400 is a terminal belonging to a person designated as the user's caregiver. It may correspond to personal mobile devices such as smartphones or tablets, but is not limited thereto. The caregiver terminal 400 may be connected to the electronic device 100 and the server 300 via a network.

Hereinafter, a blood glucose data management method according to an example embodiment of the present disclosure is described.

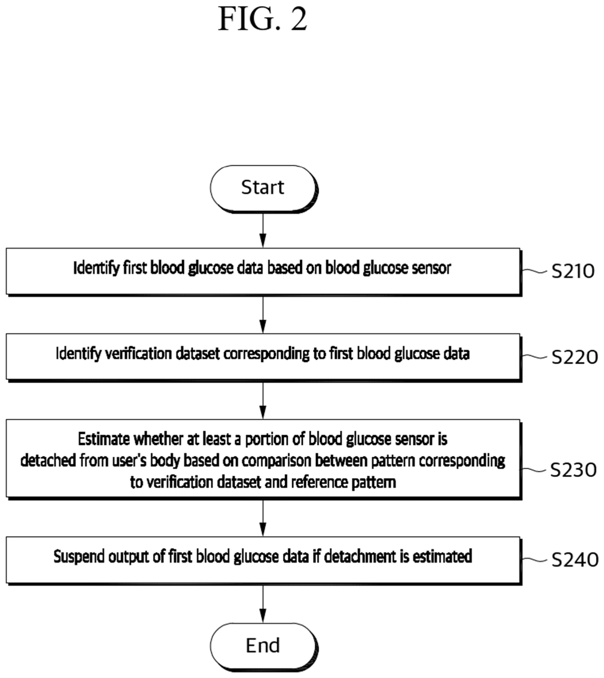

FIG. 2 is a flowchart illustrating a blood glucose data management method according to an example embodiment.

In operation S210, the electronic device 100 may identify first blood glucose data based on the blood glucose sensor 200. In operation S220, the electronic device 100 may identify a verification dataset corresponding to the first blood glucose data. In operation S230, the electronic device 100 may estimate whether at least a portion of the blood glucose sensor 200 is detached from the user's body based on a comparison between a pattern corresponding to the verification dataset and a reference pattern. In operation S240, the electronic device 100 may suspend output of the first blood glucose data to the user if detachment is estimated. The following describes each operation in detail.

First, the electronic device 100 may identify the first blood glucose data based on the blood glucose sensor 200. As described above, the blood glucose sensor 200 is percutaneously inserted into the user's body and may periodically measure the user's biometric data. An example of biometric data is the current value flowing through microelectrodes percutaneously inserted into the user's body. When such biometric data is measured, the electronic device 100 may compute the blood glucose data by processing the biometric data. For example, the electronic device 100 may perform the computation based on a mapping relationship between the biometric data and the blood glucose data, but is not limited thereto. According to an example embodiment, the first blood glucose data may correspond to data measured by the blood glucose sensor 200 at the next checking cycle of blood glucose data checked based on the biological data measured periodically by the blood glucose sensor 200 as described above. For example, the first blood glucose data may correspond to the most recent blood glucose data among the blood glucose data periodically measured based on the blood glucose sensor 200. According to another example embodiment, the first blood glucose data may correspond to any one of past blood glucose data measured periodically. However, for the sake of convenience in the following description, the description will be based on the aforementioned example embodiment, namely the example embodiment where the first blood glucose data corresponds to the most recent blood glucose data. Nevertheless, the content described below may also apply when the first blood glucose data corresponds to any one of past blood glucose data.

According to an example embodiment, the electronic device 100 may identify a verification dataset corresponding to the first blood glucose data. The verification dataset may correspond to a set containing a certain number of consecutive blood glucose data that may identify a pattern in blood glucose data. The verification dataset corresponding to the first blood glucose data may include the first blood glucose data and at least one preceding blood glucose data with a sequential blood glucose measurement order, in a time-series alignment. For example, if blood glucose data is set to be measured at 5-minute intervals, the verification dataset for the first blood glucose data measured at 13:30 on January 1, 2025, may include, along with the first blood glucose data, the blood glucose data measured at 13:25, 13:20, 13:15 on the same day, in a time-series alignment. The number of blood glucose data included in the verification dataset may be statically set or dynamically set. The following describes an example embodiment for dynamically setting the number of blood glucose data to be included in the verification dataset.

According to an example embodiment, the electronic device 100 may set the number of data in the verification dataset inversely proportional to the magnitude of the blood glucose value indicated by the first blood glucose data. For example, the electronic device 100 may set the number of data included in the verification dataset to be larger when the magnitude of the blood glucose value indicated by the first blood glucose data is small. This example embodiment has the advantage of allowing a somewhat conservative, cautious approach based on more data to distinguish it from actual hypoglycemia when the magnitude of the blood glucose value indicated by the first blood glucose data is small.

According to an example embodiment, the verification dataset may further include, in addition to the plurality of blood glucose data, error history information, operational history information, and specification information of the blood glucose sensor 200. Based on such information, example embodiments may also be applied where certain thresholds for each reference pattern, which will be described later, are adjusted.

According to an example embodiment, the electronic device 100 may identify a plurality of blood glucose data in a time-series arrangement included in the verification dataset. Furthermore, the electronic device 100 may determine whether the pattern of the plurality of blood glucose data corresponds to at least one of the first reference pattern and the second reference pattern included in the reference patterns. If the pattern of the plurality of blood glucose data corresponds to at least one of the first reference pattern and the second reference pattern, the electronic device 100 may estimate detachment of at least a portion of the blood glucose sensor 200. The first reference pattern may relate to the relative magnitude between blood glucose data, and the second reference pattern may relate to the absolute magnitude of the blood glucose data. First, the first reference pattern will be described.

For example, if a portion of the blood glucose sensor is detached, blood glucose data may be measured excessively low due to non-response caused by insufficient glucose in the air or because current cannot flow through the microelectrodes due to the air's electrical resistance being much higher than that of interstitial fluid. Based on such cases, according to an example embodiment, the first reference pattern may include a pattern where, among the plurality of blood glucose data, the subsequent blood glucose data in a sequence of consecutive blood glucose measurements decreases by at least a first threshold compared to the preceding blood glucose data. An example of the first threshold may correspond to a blood glucose decrease not typically observed even in diabetic patients, such as a value around 150 mg/dL, but is not limited to such a value.

For example, if a portion of the blood glucose sensor is detached, it can be predicted that, as mentioned earlier, blood glucose data measured excessively low will not be followed by measured blood glucose data that has risen again. Based on this observation, according to an example embodiment, the first reference pattern may further include a pattern where the slope between two blood glucose data points, measured in a later sequence than the preceding blood glucose data, is 0 or less. For example, the first reference pattern may further include a pattern where blood glucose data does not increase again after a sharp drop.

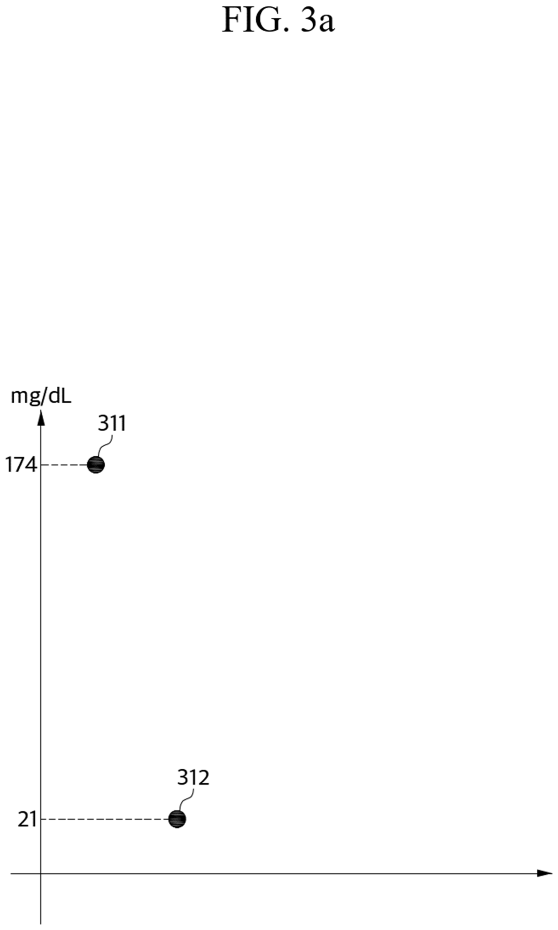

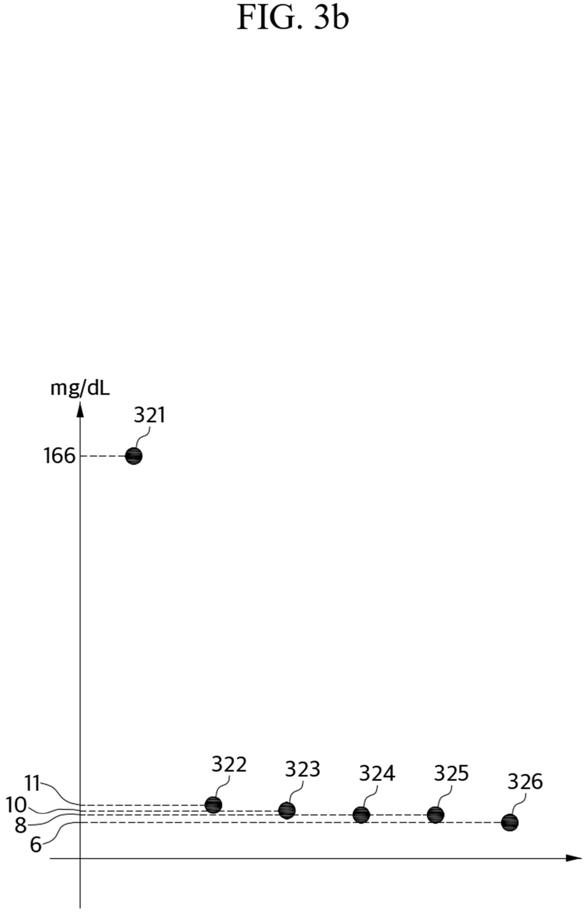

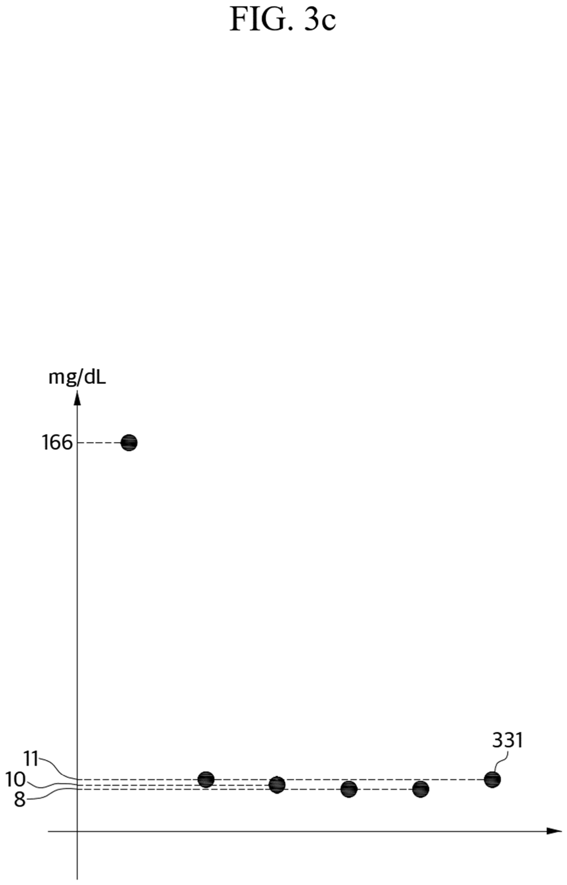

Examples where patterns in the verification dataset correspond to the first reference pattern and examples where they do not correspond are described below with reference to FIGS. 3A, 3B, and 3C.

FIG. 3A is a diagram showing an example where the pattern of a verification dataset corresponds to the first reference pattern.

Referring to FIG. 3A, an example where the blood glucose data 311 corresponded to 174 mg/dL, but the very next sequence showed the first blood glucose data 312 measured at 21 mg/dL can be seen. In such a case, the pattern corresponding to the verification dataset may be confirmed to match the first reference pattern.

FIG. 3B is a diagram showing another example where the pattern of a verification dataset corresponds to the first reference pattern.

Referring to FIG. 3B, the blood glucose data 321 corresponded to 166 mg/dL before being measured at 11 mg/dL (322). Subsequently, the blood glucose data 323, 324 and 325 continued to show a pattern of not increasing at 10 mg/dL, 8 mg/dL, and 8 mg/dL, and the first blood glucose data 326 was still measured at 6 mg/dL. In such a case, since the blood glucose data is not increasing again after a sharp drop, the pattern corresponding to the verification dataset may be confirmed to correspond to the first reference pattern.

FIG. 3C is a diagram showing an example where the pattern of a verification dataset does not correspond to the first reference pattern.

Referring to FIG. 3C, an example can be observed where the first blood glucose data 331 has increased to 11 mg/dL compared to before, generally similar to FIG. 3B but slightly different. In such a case, since the blood glucose data has increased again after a sharp drop, it can be confirmed that the pattern corresponding to the verification dataset does not match the first reference pattern.

Next, the second reference pattern will be described. According to an example embodiment, the second reference pattern may include a pattern where at least one blood glucose data among a plurality of blood glucose data is below a second threshold. Here, the second threshold may correspond to a low blood glucose value rarely measured even in diabetic patients, such as approximately 30 mg/dL, but is not limited to this value.

According to an example embodiment, the second reference pattern may further include a pattern related to the timing when at least one blood glucose data was measured below the second threshold. For example, the second reference pattern may further include a pattern where the timing when at least one blood glucose data was measured below the second threshold falls within a predetermined time range calculated from the timing when a hypoglycemia alert was output to the user. When a hypoglycemia alert is issued, it is clear that the user will take countermeasures, such as eating food to raise blood glucose, to prevent hypoglycemic shock. However, if another blood glucose data falls below the second threshold within a predetermined time range calculated from the time the hypoglycemia alert was issued, it may be more reasonable to infer that the sensor has partially detached rather than that the user's blood glucose has actually plummeted. This insight forms the basis for setting the second reference pattern as described. Here, the predetermined time range may be appropriately set as the time interval during which blood glucose is not expected to drop sharply after the user takes countermeasures.

Alternatively, the second reference pattern may further include a pattern where a reference number of blood glucose data are measured to be below the second threshold starting from the first blood glucose data measured to be below the second threshold. This may also be based on the observation that, as mentioned earlier, when a hypoglycemia alert occurs, the user will take countermeasures to raise their blood glucose. Therefore, when blood glucose remains persistently very low, it may be more reasonable to assume that the sensor has partially detached rather than infer an actual hypoglycemic state. In this case, the reference number may be set to correspond to the time when it is predicted that the user will necessarily take countermeasures to avoid hypoglycemic shock, considering the measurement cycle.

According to an example embodiment, the electronic device 100 may adjust at least one threshold related to the reference pattern based on the user's medical history information. That is, the electronic device 100 may adjust, for example, the first threshold related to the first reference pattern or the second threshold related to the second reference pattern, considering the user's medical history related to their disease. Each example will now be described in detail.

For example, in patients with type 1 diabetes, the body produces no insulin at all, leading to greater fluctuations in blood glucose levels compared to other types of diabetes. Consequently, patients with type 1 diabetes may experience rapid drops in blood glucose. In such cases, even if blood glucose data falls sharply outside the normal range, caution is needed to avoid interpreting this as sensor detachment rather than hypoglycemia. Noting this point, the electronic device 100 may identify medical history information indicating the user has type 1 diabetes. In this case, the electronic device 100 may adjust the first threshold of the first reference pattern related to the decrease amount of the subsequent blood glucose data relative to the preceding blood glucose data in a sequence of blood glucose measurements to be increased. Through such an example embodiment, sensor detachment estimation for type 1 diabetes patients may be performed conservatively.

As another example, for patients who have experienced hypoglycemic shock, caution is similarly needed to estimate sensor detachment rather than hypoglycemia even if blood glucose data is somewhat low. Therefore, the electronic device 100 may identify medical history information indicating that the user has a history of hypoglycemic shock. In this case, the electronic device 100 may adjust the second threshold of the second reference pattern related to the magnitude of at least one blood glucose data point among the plurality of blood glucose data included in the verification dataset to be reduced. Through such an example embodiment, sensor detachment estimation for patients who have experienced hypoglycemic shock may be performed conservatively. The above description regarding threshold adjustment is merely illustrative, and the scope of the present disclosure is not limited to the aforementioned examples.

According to an example embodiment, the electronic device 100 may also estimate detachment of at least a portion of the blood glucose sensor 200 based on a third reference pattern. Specifically, the electronic device 100 may perform stabilization operations after the blood glucose sensor 200 is initially inserted percutaneously into the user's body. During such a stabilization period, the current values of the biological data measured by the blood glucose sensor 200 may typically fluctuate, meaning large deviations in values may be measured across consecutive measurement sequences. Consequently, the blood glucose data that may be determined based on these current values may also fluctuate. Accordingly, the electronic device 100 may perform an operation, as at least part of the stabilization operation, of waiting until the current values are no longer fluctuating, rather than performing a process of checking blood glucose data based on such fluctuating current values or a process of outputting the blood glucose data. Therefore, when the electronic device 100 is performing the stabilization operation and the blood glucose sensor 200 is properly inserted without being detached, it may be predicted that the current values will fluctuate.

Based on this point, according to an example embodiment, the electronic device 100 may compare the pattern of a plurality of blood glucose data included in the verification dataset with the third reference pattern corresponding to the stabilization operation when it is confirmed that it is performing the stabilization operation. Here, the third reference pattern may include a pattern where the average change between two consecutive blood glucose measurement data is equal to or greater than a third threshold. For example, the electronic device 100 may identify a plurality of blood glucose data included in the verification dataset, calculate the average change between two consecutive data among them, and then determine whether this average is equal to or greater than the third threshold. If the average is equal to or greater than the third threshold, the electronic device 100 may confirm that the pattern of the plurality of blood glucose data included in the verification dataset corresponds to the third reference pattern and infer that at least a portion of the blood glucose sensor is not detached and is properly inserted into the user's body. Conversely, if the average is below the third threshold, the electronic device 100 may confirm that the pattern of the plurality of blood glucose data included in the verification dataset does not correspond to the third reference pattern. In this case, according to an example embodiment, since the pattern of the blood glucose data, which should be irregular if the blood glucose sensor 200 is properly inserted, is not found to be irregular, the electronic device 100 may infer that at least a portion of the blood glucose sensor 200 is detached.

According to an example embodiment, the electronic device 100 may estimate sensor detachment by confirming that the pattern of the verification dataset corresponding to the first blood glucose data corresponds to at least one of the first reference pattern or the second reference pattern, or by confirming that it does not correspond to the third reference pattern, and then suspend output of the first blood glucose data to the user. That is, measured blood glucose data is basically output to the user immediately, but blood glucose data estimated to have been measured from a detached sensor may have its output to the user suspended. Here, output suspension encompasses all actions that allow the user to view complete blood glucose data later than the typical checking time for general blood glucose data. For example, it may allow only partial blood glucose data to be viewed until a certain point in time, prevent any blood glucose data from being viewed until that point, allow only the range of the blood glucose value to be viewed, display it faintly on a graph so the exact value cannot be discerned, indicate it with a different icon to suggest that the data is presumed to be measured from a detached sensor, or only show the trend of data increase or decrease. Specific examples of such output suspension actions are described below, along with several actions that may be linked to them.

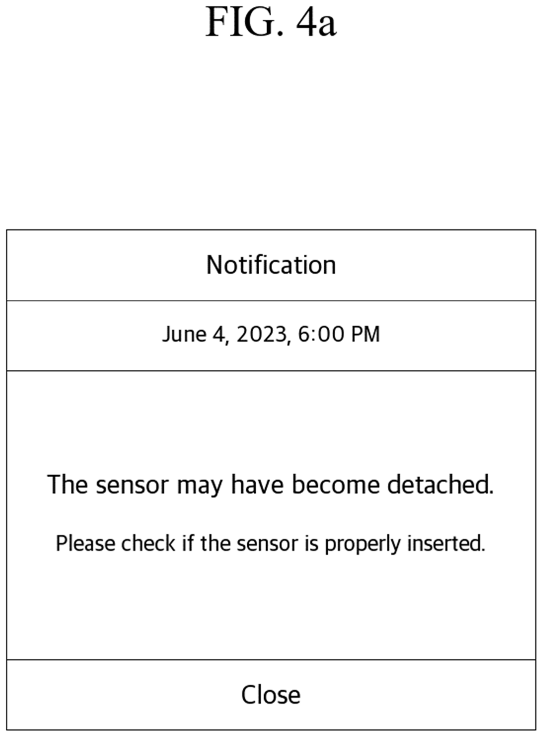

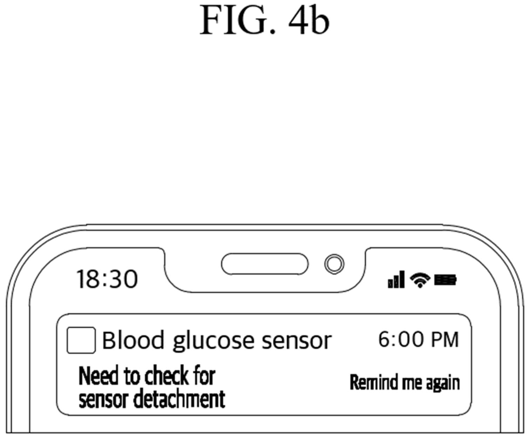

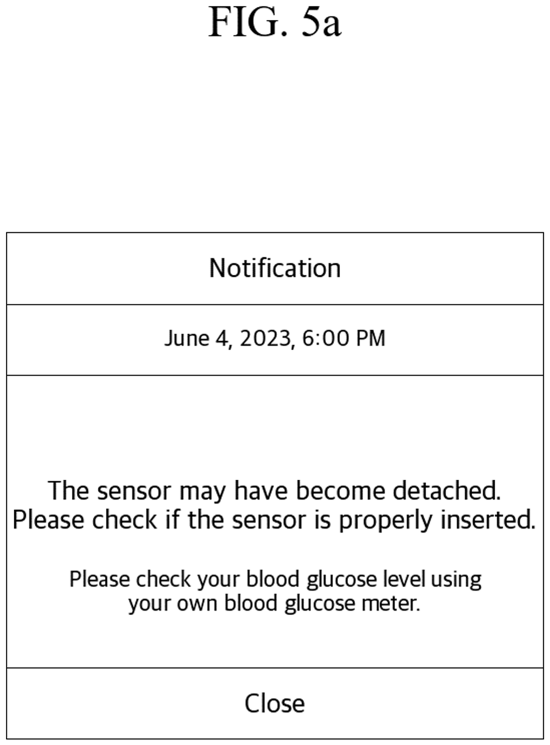

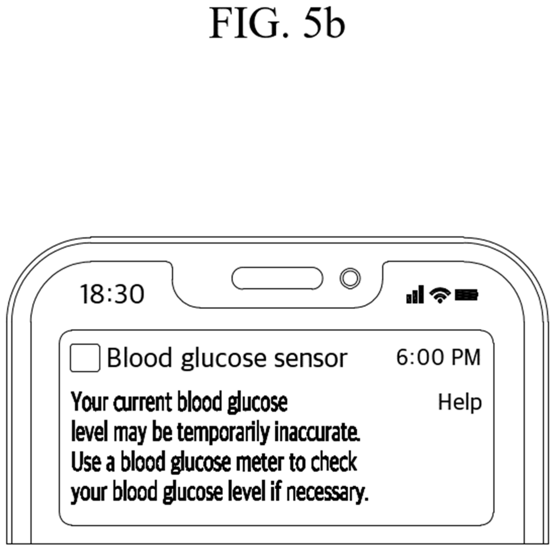

First, when the output of the first blood glucose data is suspended due to the estimated detachment of at least a portion of the blood glucose sensor 200, the electronic device 100 may output a notification related to sensor detachment to the user. The notification related to sensor detachment may include a message urging the user to carefully check the sensor, as it may have become detached. Refer to FIGS. 4A and 4B, and FIGS. 5A and 5B to examine examples of such notifications.

FIG. 4A is a diagram showing an example where a notification related to sensor detachment is displayed via a notification window according to an example embodiment.

Referring to FIG. 4A, an example of a notification related to sensor detachment displayed via a notification window on a display device operating in conjunction with the electronic device 100 can be seen. The notification related to sensor detachment emphasizes the message "The sensor may have become detached" by displaying it in a relatively large font size. Below this, the message "Please check if the sensor is properly inserted" may be displayed in a relatively smaller font size and highlighted. In cases where the notification related to sensor detachment is delivered in the form shown in FIG. 4A, according to an example embodiment, it may correspond to a situation where the user is using an application related to the blood glucose sensor 200 via the electronic device 100.

FIG. 4B is a diagram showing an example where a notification related to sensor detachment is displayed via a banner according to an example embodiment.

Referring to FIG. 4B, an example of a notification related to sensor detachment displayed via a banner on a display device operating in conjunction with an electronic device 100 can be seen. As shown in FIG. 4B, the notification related to sensor detachment provided via the banner may contain only a concise message, such as "Need to check for sensor detachment," unlike the notification provided across a large area of the screen on the display device as shown in FIG. 4A. When a sensor detachment notification is delivered in the form shown in FIG. 4B, according to an example embodiment, this may correspond to a situation where the user is not using an application related to the blood glucose sensor 200 via the electronic device 100.

FIG. 5A is a diagram showing another example where a notification related to sensor detachment is displayed via a notification window according to an example embodiment.

Referring to FIG. 5A, another example of a notification related to sensor detachment displayed via a notification window on a display device operating in conjunction with an electronic device 100 can be seen. This is similar to FIG. 4A but differs in some aspects. In the case of FIG. 5A, as a difference from FIG. 4A, it may further include a message informing the user that they will need to perform a self-test of blood glucose if they wish to check their blood glucose level, along with a message asking them to check whether the sensor is functioning properly because an abnormal sensor signal has been detected. Here, the self-test of blood glucose encompasses various methods by which the user may directly measure their own blood glucose, not using the blood glucose sensor 200. One example is the self-monitoring of blood glucose (SMBG) method using a lancet and a blood glucose meter, but it is not limited to this.

FIG. 5B is a diagram showing another example where a notification related to sensor detachment is displayed via a banner according to an example embodiment.

FIG. 5B also shows another example of a notification related to sensor detachment displayed via a banner on a display device operating in conjunction with an electronic device 100, similar to but differing in part from FIG. 4B. Specifically, FIG. 5B corresponds to the example in FIG. 5A and, similarly to FIG. 5A, it may include a message informing the user that they must perform a self-test of blood glucose if they wish to check their blood glucose level. When a sensor detachment notification is delivered in the form shown in FIG. 5B, according to an example embodiment, this may correspond to a situation where the user is not using an application related to the blood glucose sensor 200 via the electronic device 100.

Such a notification may also be delivered to the caregiver terminal 400 set up for the user. The notification for the caregiver terminal 400 may also be displayed via a notification window or banner on a display device operating in conjunction with the caregiver terminal 400, similar to FIGS. 4A and 4B, and FIGS. 5A and 5B. In this case, the message may also be partially modified to suit the needs of the caregiver operating the caregiver terminal 400, or additional messages may be included. For example, if it is estimated that the blood glucose sensor 200 has been detached for an extended period of time, the notification related to sensor detachment sent to the caregiver terminal 400 may further include a message regarding the estimated duration of detachment of the blood glucose sensor 200 and a message urging the caregiver to check it.

According to an example embodiment, the notification regarding sensor detachment may be different from a measurement notification output each time the blood glucose sensor 200 measures blood glucose, a hypoglycemia notification output when hypoglycemia is detected, a hyperglycemia notification output when hyperglycemia is detected, a rapid change notification output when blood glucose fluctuates rapidly, an anomaly notification output when other sensor abnormalities are detected, and so forth, in terms of at least some of various parameters related to the notifications, such as sound volume, vibration intensity, and notification type. For example, compared to urgent alerts like hypoglycemia or rapid change notifications, the sound volume or vibration intensity may be relatively lower, while compared to routine alerts like measurement notifications, the sound volume or vibration intensity may be relatively higher. Additionally, regarding notification type, a custom melody or vibration pattern may be applied exclusively to notification regarding sensor detachment, enabling the user to immediately recognize that the notification pertains to sensor detachment.

According to an example embodiment, in a situation where output-suspended blood glucose data exists, a request for output of the blood glucose graph may be identified from the user. Specifically, the request for output of the blood glucose graph may be identified through user input via an application related to the blood glucose sensor 200 operating on the electronic device 100. In this case, the electronic device 100 may deactivate the portion of the blood glucose graph corresponding to the blood glucose data whose output has been suspended. As described above, if the output of the first blood glucose data is suspended, the deactivated portion may include the portion corresponding to the first blood glucose data. After such processing, the electronic device 100 may output the blood glucose graph, with the portion corresponding to the output-suspended blood glucose data deactivated, to a display device linked to the electronic device 100. According to an example embodiment, the blood glucose graph may be a graph where the horizontal axis corresponds to the time axis, the vertical axis corresponds to the magnitude of blood glucose values, and each point on the graph represents each blood glucose data measured at a specific time point.

Here, deactivation processing may include, according to an example embodiment, at least some of blurring processing for points on the graph, non-responsiveness processing to clicks, and display processing related to output suspension indication. For example, the blurring processing may include processing to display points on the graph corresponding to output-suspended data in a blurred state. For example, the non-responsiveness processing to clicks may include processing to ensure that clicking on output-suspended points does not display additional information, unlike regular points where blood glucose data is displayed in detail upon clicking. For example, display processing related to output suspension indication may include displaying a message indicating that these points correspond to output-suspended data and thus have undergone blurring or non-responsiveness processing. Furthermore, deactivation processing may, according to an example embodiment, include displaying blood glucose data using an icon different from other points on the graph. For instance, distinct icons could be used to display blood glucose data presumed not to be acquired from a detached sensor and blood glucose data presumed to be acquired from a detached sensor on the blood glucose graph, enabling clear differentiation between the two. As an additional example, deactivation processing may include processing to prevent any UI/UX feedback from being displayed even when a click is input for coordinates on the graph corresponding to output-suspended data. When applying such deactivation processing, the electronic device 100 may display appropriate feedback when a click is input for coordinates on the graph corresponding to data not output-suspended, but may display no feedback for output-suspended data. This allows the user to recognize that clicks are not possible for output-suspended data. The deactivation processing is not limited to the above examples and may encompass any processing that allows the user to view complete blood glucose data later than the time when normal blood glucose data is typically viewed, for output-suspended data.

Such blood glucose graphs may also be requested via the caregiver terminal 400. In this case, the caregiver terminal 400 may obtain the overall blood glucose data through linkage with the server 200 or the electronic device 100, and based on this, display the blood glucose graph on a display device linked to the caregiver terminal 400. At this time, if there is output-suspended blood glucose data, that portion may be displayed as inactive, similar to the aforementioned method.

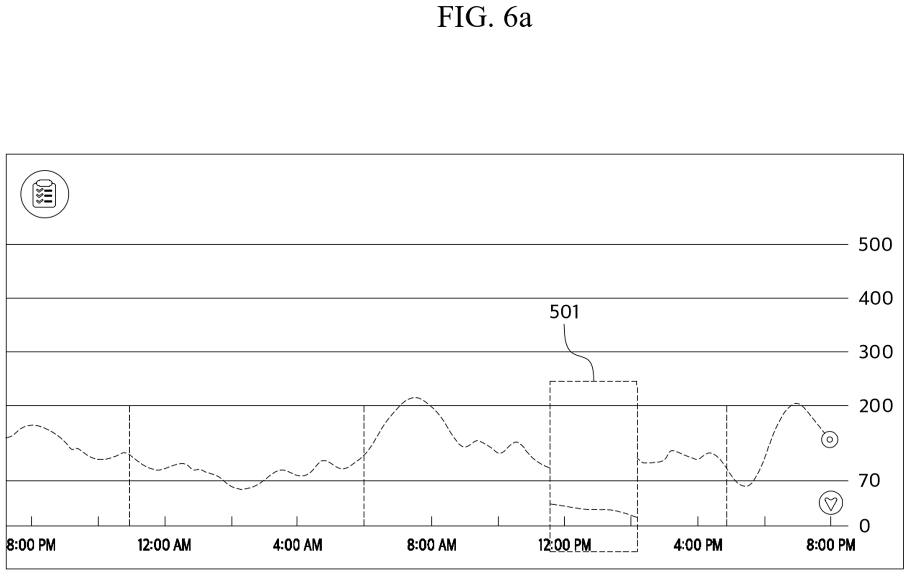

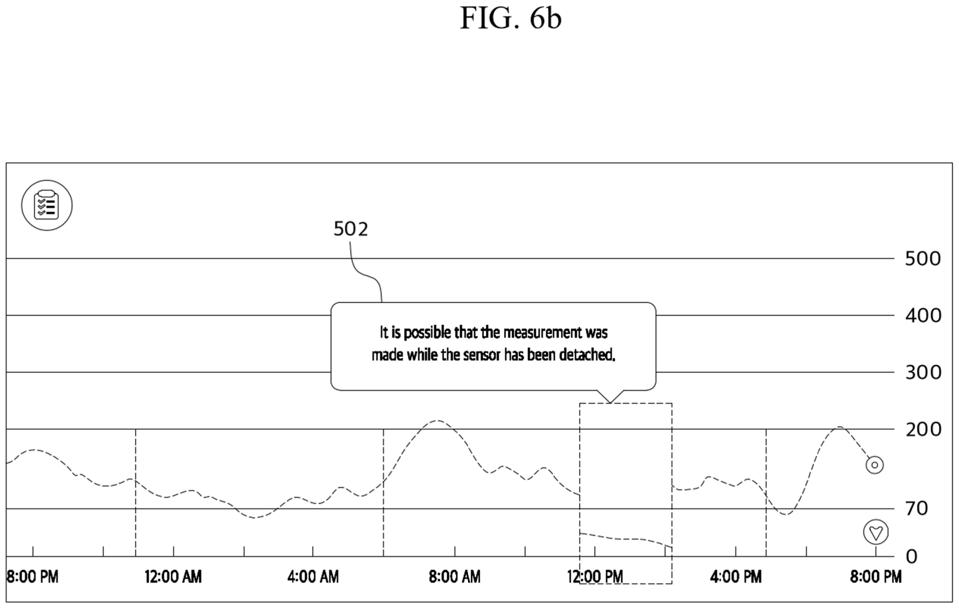

Refer to FIGS. 6A and 6B to examine an example of a blood glucose graph where output-suspended blood glucose data has been deactivated.

FIG. 6A is an example diagram of a blood glucose graph where blood glucose data whose output has been suspended are deactivated according to an example embodiment.

Referring to FIG. 6A, it can be observed that the corresponding points for the values 501, which are presumed to be blood glucose data measured while the blood glucose sensor 200 is detached, are displayed more faintly compared to other points on the blood glucose graph. Thus, the values 501 presumed to be blood glucose data measured while the blood glucose sensor 200 is detached may be displayed on the blood glucose graph to be distinguishable from other points.

FIG. 6B is an example diagram showing a UI when the blood glucose data whose output has been suspended and thus deactivated is clicked according to an example embodiment.

Referring to FIG. 6B, an example notification 502 that may be displayed when the user selects the value 501 of the aforementioned FIG. 6A, which is presumed to be blood glucose data measured while the blood glucose sensor 200 is detached, can be seen. As shown in FIG. 6B, clearly indicating that the value may have been measured while the sensor was detached may enhance the user experience.

The above examples describe situations where information related to blood glucose is requested in a two-dimensional blood glucose graph format when the information related to blood glucose is requested by the user or caregiver. However, this is merely an example, and the information related to blood glucose may be requested in formats other than graphs, such as tabular logs, distribution charts, radial charts, calendar maps, cumulative area charts, box plots, and other formats. It is not limited to the examples listed above. The electronic device 100 may provide the user or caregiver with the information related to blood glucose in various formats. Regardless of the format in which the information related to blood glucose is provided, various processing methods, such as the deactivation processing according to output suspension described above, may be similarly applied to blood glucose data presumed to be measured after sensor detachment, and these are also considered to be included in the scope of the present disclosure.

According to an example embodiment, the electronic device 100 may estimate sensor detachment by confirming that the pattern of the verification dataset corresponding to the first blood glucose data corresponds to at least one of the first reference pattern or the second reference pattern, and then check the second blood glucose data based on the blood glucose sensor 200 in the next measurement cycle. If the second blood glucose data is equal to or smaller than the first blood glucose data, the electronic device 100 may compare the pattern of the verification dataset corresponding to the second blood glucose data with the first and second reference patterns to re-estimate whether the sensor is detached. However, if the second blood glucose data is greater than the first blood glucose data, the electronic device 100 may confirm that the first blood glucose data was not measured in a state that the sensor has been detached. Specifically, as described above, if the blood glucose sensor 200 had actually detached, it may be assumed that the blood glucose data would not increase again. Therefore, when the second blood glucose data is larger in magnitude than the first blood glucose data, it may be assumed that the blood glucose sensor 200 had not actually been detached. From this perspective, the electronic device 100 may assume that the first blood glucose data was not measured in a state that the sensor has been detached, and thus may release the suspension on outputting the first blood glucose data. Accordingly, the electronic device 100 may output the first blood glucose data to the user normally. That is, it may release the deactivation processing applied on the blood glucose graph and display the blood glucose data identically to regular points.

According to an example embodiment, a flag related to compression noise may be selectively set for data whose output is suspended, such as the first blood glucose data. For example, since applying pressure to the blood glucose sensor 200 may sometimes result in relatively low measurement values, the flag related to compression noise may be set for the data suspected of having low values due to this issue. Such compression noise flags may be set based on data from a pressure sensor installed alongside the blood glucose sensor 200, or set for data collected during time periods where compression frequently occurs due to tossing and turning during sleep. Alternatively, an example embodiment is also possible in which the flag related to compression noise is set by default for all data whose outputs are suspended.

If it is confirmed that the flag related to compression noise is set for the first blood glucose data, the electronic device 100 may control the blood glucose sensor 200 to measure the second blood glucose data earlier than the default cycle. That is, if the default cycle for measuring blood glucose data is 5 minutes, the electronic device 100 may control the blood glucose sensor 200 to measure the second blood glucose data again in just 1 minute. Since the pressure applied to the blood glucose sensor 200 is typically relieved soon, the second blood glucose data will be measured normally, i.e., higher than the first blood glucose data measured low due to compression noise. In such a case, the electronic device 100 may release the output suspension of the first blood glucose data, but output a notification to the user that includes a message indicating that the first blood glucose data is presumed to be low due to compression noise. Alternatively, instead of the first blood glucose data, the electronic device 100 may selectively perform interpolation or extrapolation using previously measured blood glucose data and the second blood glucose data to output adjusted data to the user. For example, the electronic device 100 may estimate the accurate blood glucose value at the time of the first blood glucose data measurement by performing interpolation based on the second blood glucose data, which was measured normally without compression noise, and the most recent blood glucose data measured prior to the measurement of the first blood glucose data, instead of the first blood glucose data. As another example, the electronic device 100 may estimate the accurate blood glucose value at the time of the first blood glucose data measurement by performing extrapolation using the most recent blood glucose data measured before the first blood glucose data was measured or a plurality of blood glucose data measured after the first blood glucose data was measured.

According to an example embodiment, the electronic device 100 may determine whether the number of consecutive blood glucose data whose output has been suspended corresponds to a first reference number. If the number of consecutive blood glucose data whose output has been suspended corresponds to the first reference number, the electronic device 100 may disconnect from the blood glucose sensor 200. Here, the first reference number may be appropriately set as the number of data which may reliably be estimated that the blood glucose sensor 200 has been detached, considering the measurement cycle. After disconnecting from the blood glucose sensor 200, the electronic device 100 may output a notification to the user containing a message requesting connection of a new blood glucose sensor since the connection to the blood glucose sensor has been disconnected.

According to an example embodiment, the electronic device 100 may identify a new blood glucose sensor that has replaced the blood glucose sensor 200 following disconnection from the blood glucose sensor 200. Then, the electronic device 100 may obtain blood glucose data based on the new blood glucose sensor. The electronic device 100 may determine estimated blood glucose data for at least some of the blood glucose data whose output has been suspended by performing interpolation or extrapolation using the blood glucose data identified based on the new blood glucose sensor. For example, the electronic device 100 may determine estimated blood glucose data for blood glucose data measured at a point in time between the two points but whose output was suspended, by performing interpolation using the blood glucose data identified based on the new blood glucose sensor and the most recent blood glucose data among the blood glucose data whose output was not suspended. Alternatively, the electronic device 100 may determine estimated blood glucose data for blood glucose data measured at a point in time prior to the point in time at which measurement was performed by the new blood glucose sensor but whose output was suspended, by performing extrapolation using a plurality of blood glucose data identified based on the new blood glucose sensor. The electronic device 100 may output the estimated blood glucose data thus determined to the user. For example, upon receiving a request to output a blood glucose graph, the electronic device 100 may display at least some of the data whose output was suspended on the blood glucose graph, replacing them with the estimated blood glucose data as described above, and provide it to the user.

The various operations of the electronic device 100 described above may also be configured to be performed in conjunction with the server 300. For example, the electronic device 100 may be configured to perform the role of transmitting data from the blood glucose sensor 200 to the server 300. In this case, the server 300 may perform the various operations described above on behalf of the electronic device 100 based on the transmitted data. In this case, when a request for the various pieces of information is input from the user via an application operating on the electronic device 100, the server 300 may transmit the various pieces of information determined through operations to the electronic device 100 for output to the user.

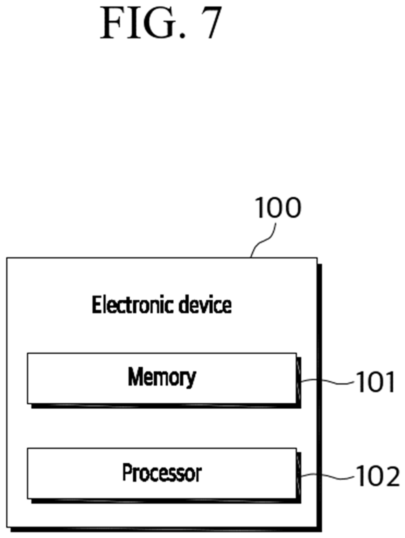

FIG. 7 shows a block diagram of an electronic device according to an example embodiment.

The electronic device 100 may include a memory 101 and a processor 102 according to an example embodiment. The electronic device 100 shown in FIG. 7 depicts only the components related to this example embodiment. Therefore, it will be understood by those skilled in the art related to this example embodiment that other common components may be included in addition to the components illustrated in FIG. 7. In an example embodiment, the processor 102 may be included in a controller.

The processor 102 may control the overall operation of the electronic device 100 and process data and signals. The processor 102 may be composed of at least one hardware unit. Furthermore, the processor 102 may operate based on one or more software modules generated by executing program code stored in the memory 101. The processor 102 may include a memory, in which the processor 101 may execute program code stored in the memory to control the overall operation of the electronic device 100 and process data and signals.

The processor 102 may be configured to identify first blood glucose data based on the blood glucose sensor, identify a verification dataset corresponding to the first blood glucose data, estimate whether at least a portion of the blood glucose sensor is detached from a user's body based on a comparison between a pattern corresponding to the verification dataset and a reference pattern, and suspend output of the first blood glucose data for the user if detachment is estimated.

Depending on example embodiments, the electronic device 100 may further include a transceiver for performing wired/wireless communication. The electronic device 100 may communicate with external electronic devices using the transceiver. The external electronic devices may be terminals or servers. Furthermore, the communication technologies utilized by the transceiver may include GSM(Global System for Mobile communication), CDMA (Code Division Multi Access), LTE(Long Term Evolution), 5G, WLAN(Wireless LAN), Wi-Fi(Wireless-Fidelity), Bluetooth^{™}, RFID(Radio Frequency Identification), IrDA (Infrared Data Association), ZigBee, NFC(Near Field Communication), etc.

According to example embodiments, one or more of the following effects can be expected.

According to the example embodiments of this specification, detachment of a blood glucose sensor may be detected based on blood glucose patterns.

Furthermore, according to the example embodiments of this specification, blood glucose data obtained from a detached sensor may be managed.

Furthermore, according to the example embodiments of this specification, detachment of a blood glucose sensor may be notified to the user in various ways.

The effects of the present disclosure are not limited to the effects mentioned above, and other effects not mentioned will be clearly understood by those skilled in the art from the description of the claims.

The electronic device according to the above-described example embodiments may include a processor, a memory for storing and executing program data, a permanent storage such as a disk drive, a communication port for communicating with an external device, a user interface device such as a touch panel, a key, a button, or the like. Methods implemented as software modules or algorithms may be stored on a computer-readable recording medium as computer-readable codes or program instructions executable on the processor. Here, the computer-readable recording medium includes a magnetic storage medium (e.g., ROM (read-only memory), RAM (random-access memory), floppy disk, hard disk, etc.) and optical reading medium (e.g., CD-ROM and DVD (Digital Versatile Disc)). The computer-readable recording medium is distributed over networked computer systems, so that computer-readable codes can be stored and executed in a distributed manner. The medium is readable by a computer, stored in a memory, and executed on a processor.

The present example embodiment can be represented by functional block configurations and various processing steps. These functional blocks may be implemented with various numbers of hardware or/and software configurations that perform specific functions. For example, the example embodiment may employ an integrated circuit configuration such as memory, processing, logic, look-up table, or the like, capable of executing various functions by control of one or more microprocessors or other control devices. Similar to that components can be implemented with software programming or software elements, this example embodiment includes various algorithms implemented with a combination of data structures, processes, routines or other programming components and may be implemented with a programming or scripting language including C, C++, Java, assembler, etc. Functional aspects can be implemented with an algorithm running on one or more processors. In addition, the present example embodiment may employ a conventional technique for at least one of electronic environment setting, signal processing, and data processing. Terms such as "mechanism", "element", "means", and "composition" can be used in a broad sense, and are not limited to mechanical and physical configurations. Those terms may include the meaning of a series of routines of software in connection with a processor or the like.

The above-described example embodiments are merely examples, and other example embodiments may be implemented within the scope of the claims to be described later.

## Claims

1. A blood glucose data management method of an electronic device (100), the blood glucose data management method comprising:
identifying first blood glucose data based on a blood glucose sensor (200);
identifying a verification dataset corresponding to the first blood glucose data;
estimating whether at least a portion of the blood glucose sensor (200) is detached from a user's body based on a comparison between a pattern corresponding to the verification dataset and a reference pattern; and
suspending output of the first blood glucose data if detachment is estimated.

2. The blood glucose data management method of claim 1, further comprising:
before the identifying of the first blood glucose data based on the blood glucose sensor, checking blood glucose data periodically based on the blood glucose sensor (200) inserted into the user's body,
wherein the first blood glucose data is measured by the blood glucose sensor (200) during the next measurement cycle of the blood glucose data that is periodically checked.

3. The blood glucose data management method of claim 1 or 2, wherein the identifying of the verification dataset corresponding to the first blood glucose data comprises:
identifying at least one preceding blood glucose data whose blood glucose measurement sequence is consecutive to the first blood glucose data; and
identifying the verification dataset including the first blood glucose data and the at least one preceding blood glucose data in a time-series alignment.

4. The blood glucose data management method of any one of claims 1 to 3, wherein the estimating of whether at least a portion of the blood glucose sensor (200) is detached comprises:
identifying a plurality of time-series aligned blood glucose data included in the verification dataset;
determining whether a pattern of the plurality of blood glucose data corresponds to at least one of a first reference pattern and a second reference pattern included in the reference pattern; and
estimating detachment of at least a portion of the blood glucose sensor (200) if the pattern of the plurality of blood glucose data corresponds to at least one of the first and second reference patterns.

5. The blood glucose data management method of claim 4, wherein the first reference pattern comprises a pattern in which, in two blood glucose data points with consecutive blood glucose measurement orders among the plurality of blood glucose data, the subsequent blood glucose data decreases by more than a first threshold compared to the preceding blood glucose data.

6. The blood glucose data management method of claim 5, wherein the first reference pattern further comprises a pattern in which the slope between two blood glucose data points whose blood glucose measurement orders are later than that of the preceding blood glucose data among the plurality of blood glucose data is less than 0.

7. The blood glucose data management method of any one of claims 4 to 6, wherein the second reference pattern comprises a pattern in which at least one blood glucose data among the plurality of blood glucose data is equal to or less than a second threshold.

8. The blood glucose data management method of any one of claims 1 to 7, further comprising:
identifying second blood glucose data that has increased compared to the first blood glucose data based on the blood glucose sensor (200);
releasing the suspension in outputting the first blood glucose data; and
outputting the first blood glucose data to the user normally.

9. The blood glucose data management method of any one of claims 1 to 8, further comprising:
determining whether the number of consecutive blood glucose data whose output has been suspended corresponds to a first reference number; and
disconnecting the connection to the blood glucose sensor (200) if the number of consecutive blood glucose data whose output has been suspended corresponds to the first reference number.

10. The blood glucose data management method of any one of claims 1 to 9, further comprising:
outputting a notification regarding sensor detachment to the user.

11. The blood glucose data management method of claim 10, wherein the outputting of the notification regarding sensor detachment to the user comprises:
identifying a terminal of the user's caregiver (400); and
outputting the notification related to sensor detachment to the terminal of the user's caregiver (400).

12. The blood glucose data management method of claim 10 or 11, wherein the notification regarding sensor detachment is different from at least a portion of a measurement notification, a hypoglycemia notification, and a hyperglycemia notification of the blood glucose sensor in terms of at least a portion of the sound volume, vibration intensity, and notification type.

13. The blood glucose data management method of any one of claims 1 to 12, further comprising:
identifying a request for output of a blood glucose graph by the user;
deactivating a portion of the blood glucose graph corresponding to blood glucose data whose output is suspended, including the first blood glucose data; and
outputting the blood glucose graph to the user, with the portion corresponding to the blood glucose data whose output is suspended being deactivated,
wherein the deactivation comprises at least a portion of blurring of points on the blood glucose graph, non-responsiveness to clicks, and displaying indication of output suspension.

14. A non-transitory computer-readable recording medium having a program for executing the blood glucose data management method of any one of claims 1 to 13 on a computer recorded thereon.

15. An electronic device (100) for managing blood glucose data, the electronic device comprising:
a processor (101); and
a memory (102) storing one or more instructions,
wherein the processor (101) is, by performing the one or more instructions, configured to:
identify first blood glucose data based on a blood glucose sensor (200);
identify a verification dataset corresponding to the first blood glucose data;
estimate whether at least a portion of the blood glucose sensor (200) is detached from a user's body based on a comparison between a pattern corresponding to the verification dataset and a reference pattern; and
suspend output of the first blood glucose data for the user if detachment is estimated.
